# EUROPEAN PATENT SPECIFICATION

(11) **EP 2 819 676 B1**
(45) Date of publication and mention of the grant of the patent: **30.05.2018**
(21) Application number: 13708635.1
(22) Date of filing: 28.02.2013
(51) Int. Cl.: A61K 31/568, A61K 31/138, A61K 45/06

(54) **COMBINATION THERAPY FOR TREATING ANDROGEN DEFICIENCY**
KOMBINATIONSTHERAPIE ZUR BEHANDLUNG VON ANDROGENMANGEL
POLYTHÉRAPIE POUR LE TRAITEMENT D'UNE CARENCE EN ANDROGÈNE

(30) Priority: 29.02.2012 US 201261604989 P
(43) Date of publication of application: 07.01.2015
(73) Proprietor: Repros Therapeutics Inc., The Woodlands, TX 77380 (US)
(72) Inventor: PODOLSKI, Joseph, S., The Woodlands, TX 77381 (US); WIEHLE, Ronald, D., Houston, TX 77059 (US)
(74) Representative: Nederlandsch Octrooibureau
(86) International application number: PCT/US2013/028356
(87) International publication number: WO 2013/130832

(56) References cited:
- WO-A1-2009/051908
- WO-A1-2013/020215
- WO-A2-02/30355
- WO-A2-2006/102232
- US-A1- 2010 111 901
- US-A1- 2010 144 687
- R. S. TAN ET AL: "An unusual case of vascular hypogonadism treated with clomiphene citrate and testosterone replacement", ANDROLOGIA, vol. 41, no. 1, 1 February 2009 (2009-02-01), pages 63-65, XP055058581, ISSN: 0303-4569, DOI: 10.1111/j.1439-0272.2008.00891.x
- BANDHAUER K ET AL: "Varicocele: spermiogram, testicular biopsy, plasma testosterone. Results of therapy", UROLOGE - AUSGABE A 1977 DE, vol. 16, no. 3, 1977, pages 154-157, XP008161214, ISSN: 0340-2592
- NICHOLSON TRISTAN ET AL: "MP48-05 COMBINATION THERAPY WITH AN AROMATASE INHIBITOR IS NEEDED IN ONE OUT OF SIX HYPOGONADAL MEN TREATED WITH CLOMIPHENE CITRATE", JOURNAL OF UROLOGY, vol. 191, no. 4, 19 May 2014 (2014-05-19), XP028603991, ISSN: 0022-5347, DOI: 10.1016/J.JURO.2014.02.1479

## Description

The present disclosure relates to a combination therapy wherein an antiestrogen or a pharmaceutically acceptable salt thereof is co-administered with an additional pharmaceutically active agent selected from an androgen and an aromatase inhibitor in order to elevate testosterone levels and/or to treat disorders related to testosterone deficiency. The present disclosure also relates to a kit comprising an antiestrogen or a pharmaceutically acceptable salt thereof with exogenous testosterone and/or one or more aromatase inhibitors. The present invention relates to a combination of trans-clomiphene or a pharmaceutically acceptable salt thereof and an aromatase inhibitor which is selected from anastrozole, letrozole and exemestane, for use in treating secondary hypogonadism or a symptom thereof in a human male with secondary hypogonadism and a body mass index of at least 30. The present invention also relates to a kit comprising an aromatase inhibitor which is selected from anastrozole, letrozole and exemestane and trans-clomiphene or a pharmaceutically acceptable salt thereof.

### BACKGROUND

Testosterone is the primary male androgen, playing a vital role in overall male health. Testosterone is essential to the development and maintenance of specific reproductive tissues (testes, prostate, epididymis, seminal vesicle, and penis) and male secondary sex characteristics. It plays a key role in libido and erectile function and is necessary for the initiation and maintenance of spermatogenesis. Testosterone also has important functions not related to reproductive tissues. For example, it positively affects body composition by increasing nitrogen retention, which supports lean body mass, muscle size and strength. It also acts on bone to stimulate bone formation.

Testosterone secretion is the end product of a series of hormonal processes. Gonadotropin-releasing hormone (GnRH), which is secreted in the hypothalamus, controls the pulsatile secretion of luteinizing hormone (LH) and follicle stimulating hormone (FSH), which are secreted by the anterior pituitary. LH, in turn, regulates the production and secretion of testosterone in the Leydig cells of the testes, while FSH assists in inducing spermatogenesis.

Testosterone is most often measured as "total testosterone." This measurement includes testosterone that is bound to sex hormone-binding globulin (SHBG) (∼44%) and is therefore not bioavailable and testosterone which either is free (∼2%) or loosely bound to other proteins (non-SHBG-bound) (∼54%).

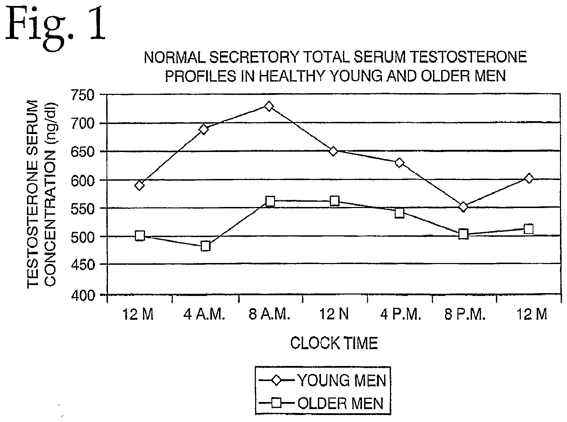

Results from a WHO study indicate that testosterone is normally secreted in a circadian rhythm, with higher levels in the morning and nadir levels occurring around 8 to 10 p.m. *See* FIG. 1. This variation in testosterone secretion throughout the day becomes much less pronounced in older men (mean age equals 71 years). The importance of this rhythm is not known at this time.

Samples were obtained from both young and elderly patients every 10 minutes for 24 hours via an indwelling cannula. According to Tenover (1987) the mean 24 hr total serum testosterone levels in healthy young men (age range 22 yrs.-35 yrs. mean 27.3 yrs) was 4.9 ± 0.3 (±SEM) mg/ml (17.0 nmol/L) while older men (age range 65yrs - 84 yrs. mean 70.7 yrs.) had a significantly lower mean 24 hrs. total serum testosterone level of 4.1 ± 0.4 mg/ml. (P < 0.5; 14.2 nmol/L).

Total serum testosterone levels obtained from single random samples were also significantly lower in older men (4.0 ± 0.2 mg/ml [13.9 n nmol/L]) as compared to 4.8 ± 0.2 mg/ml [16.6 nmol/L] in healthy young men.

Testosterone deficiency can result from underlying disease or genetic disorders and is also frequently a complication of aging. For example, primary hypogonadism results from primary testicular failure. In this situation, testosterone levels are low and levels of pituitary gonadotropins (LH and FSH) are elevated. Secondary hypogonadism is due to inadequate secretion of the pituitary gonadotropins. In addition to a low testosterone level, LH and FSH levels are low or low-normal. Some of the sequelae of adult testosterone deficiency include a wide variety of symptoms including: loss of libido, erectile dysfunction, oligospermia or azoospermia, absence or regression of secondary sexual characteristics, progressive decrease in muscle mass, fatigue, depressed mood and increased risk of osteoporosis. Many of these disorders are generically referred to as male menopause.

Several forms of testosterone therapy are presently available. Recently, transdermal preparations have gained favor in the market. However, exogenous administration of androgens (e.g. testosterone) has the effect of suppressing secretion of pituitary gonadotropins and therefore can significantly reduce sperm count. In particular, exogenous administration of testosterone leads to an inhibition of endogenous testosterone release through feedback inhibition of pituitary LH. For the same reason, spermatogenesis may also be suppressed through feedback inhibition of pituitary FSH. Accordingly, there is a need for a therapy which retains the benefits of exogenous testosterone administration while reducing or even minimizing adverse effects on male fertility.

### SUMMARY OF THE INVENTION

The present disclosure provides a combination therapy for achieving therapeutic testosterone levels in a male mammal by co-administering an antiestrogen or pharmaceutically acceptable salt thereof with an additional pharmaceutically active agent selected from an androgen and an aromatase inhibitor. In several embodiments, a method is provided for maintaining or even improving fertility parameters (e.g. sperm count, sperm morphology and/or sperm motility) in a male undergoing androgen replacement therapy in which an antiestrogen or a pharmaceutically acceptable salt thereof is co-administered with an androgen, preferably testosterone. In other embodiments, the antiestrogen or salt thereof is co-administered with an aromatase inhibitor to a male mammal in order to achieve therapeutic testosterone levels. In an embodiment of the invention, trans-clomiphene is the antiestrogen to be co-administered with the additional therapeutic agent.

The antiestrogen or pharmaceutically acceptable salt thereof may be administered simultaneously (separately or in the same formulation) or sequentially with exogenous androgen or an aromatase inhibitor to the male in order to achieve a pharmacologically effective blood androgen concentration in the male.

The combination therapy according to the disclosure may be administered to any male with low or low-normal testosterone levels including males diagnosed with or identified as likely to develop one or more of the following disorders: secondary hypogonadism, type 2 diabetes, metabolic syndrome, infertility, or osteoporosis. In a preferred embodiment of the invention, the combination therapy is administered to a human male with secondary hypogonadism.

Also provided in the disclosure is a pharmaceutical composition comprising a therapeutically effective amount of an antiestrogen or a pharmaceutically acceptable salt thereof and an androgen or an aromatase inhibitor. A kit comprising the pharmaceutical composition and instructions for use according to any method herein described is also provided.

### BRIEF DESCRIPTION OF THE DRAWING

FIG. 1 is a graphic representative of the normal secretory total serum testosterone profiles in healthy men (young and old).

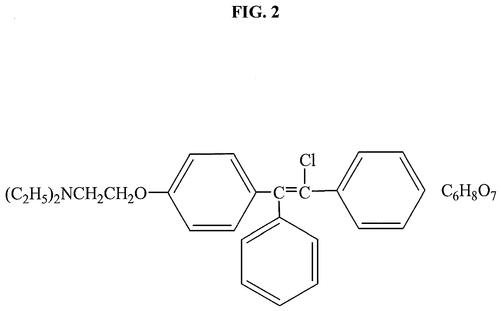

FIG. 2 shows the chemical structure of clomiphene citrate.
FIG. 3 is a graphic demonstration of the time course of serum testosterone levels with Clomid, Enclomid and Zuclomid.

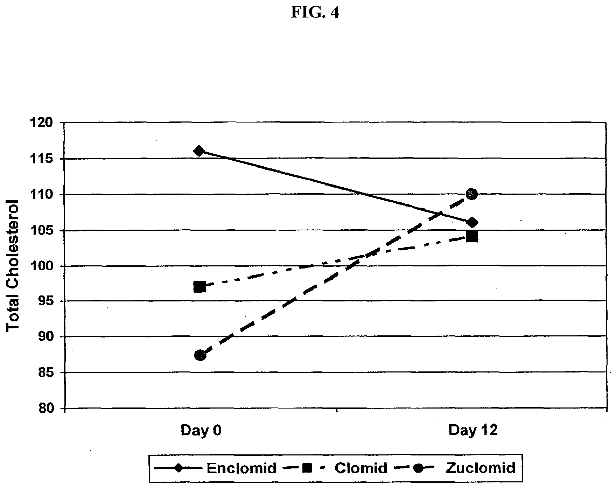

FIG. 4 is a graphic demonstration of the time course of cholesterol levels in baboon males treated with Clomid, Enclomid and Zuclomid.
FIG. 5 demonstrates the effect of Androxal™ or Androgel® on testosterone levels.
FIG. 6 demonstrates the effect of Androxal™ or Androgel® on LH levels.
FIG. 7 demonstrates the effect of Androxal™ or Androgel® on FSH levels.
FIG. 8 demonstrates the effect of Androxal or Androgel over a 7 week period (6 weeks treatment + one week follow-up) on testosterone levels.

### DETAILED DESCRIPTION

In one aspect, the present disclosure provides a combination therapy for increasing testosterone levels in male mammals and for ameliorating or preventing the symptoms of low testosterone levels, while maintaining fertility in the male by superposing administration of an antiestrogen (or salt thereof) on androgen replacement therapy in order to maintain at least one fertility parameter in the male during the therapy. In one aspect, the present invention provides a combination trans-clomiphene or a pharmaceutically acceptable salt thereof, and an aromatase inhibitor which is selected from anastrozole, letrozole and exemestane, for use in treating secondary hypogonadism or a symptom thereof in a human male with secondary hypogonadism and a body mass index of at least 30. Androgens (e.g. testosterone), when exogenously administered, act to suppress the secretion of the pituitary gonadotropins LH and FSH, thereby shutting down or greatly decreasing endogenous testosterone production and spermatogenesis. Antiestrogens (e.g. trans-clomiphene) on the other hand, exert their effect by blocking the negative feedback exerted by normal estrogens on the pituitary leading to increased secretion of these pituitary gonadotropins. This aspect of the present invention is based on the surprising discovery that co-administration of an antiestrogen with an androgen can reverse the suppression of pituitary hormones that occurs with androgen replacement therapy thereby maintaining endogenous FSH (and LH) at levels significantly above those that occur in the absence of the antiestrogen. Accordingly, this aspect of the present disclosure provides a method for maintaining spermatogenesis during androgen replacement therapy and possibly achieving a greater increase in testosterone levels than that which occurs for either agent alone. Preferably, sperm counts are maintained above 20 million/ml in the male undergoing combination therapy for a substantial portion of the co-administration period.

In another aspect, the present disclosure provides a combination therapy for increasing testosterone levels in male mammals and for ameliorating or preventing the symptoms of low testosterone levels, whereby one or more aromatase inhibitors are co-administered with an antiestrogen or pharmaceutically acceptable salt thereof. It has surprisingly been discovered that significant elevations in the estrogen level of a subset of male mammals with secondary hypogonadism, namely those with a body mass index (BMI) of at least 30, can occur during antiestrogen therapy. A weak positive correlation was observed between the degree of estrogen elevation and BMI. Aromatase is a cytochrome P-450 enzyme complex that catalyzes the rate-limiting step in the conversion of androgens (e.g. testosterone) to estrogens. Without being bound by theory it is believed that men with a BMI of at least 30 have a higher level of aromatase and therefore, as testosterone levels increase during antiestrogen therapy, a greater percentage of the newly synthesized testosterone is converted to estrogen. Accordingly, co-administration of an aromatase inhibitor with an antiestrogen provides a method for preventing or ameliorating the increase in estrogen levels in male mammals which may accompany antiestrogen therapy. In a preferred embodiment of the invention, the antiestrogen or salt thereof is trans-clomiphene and the male mammal is a secondary hypogonadal male with a body mass index (BMI) of at least 30.

"Co-administration" or "combination therapy" means administration of an antiestrogen and an androgen or an aromatase inhibitor in a sequential manner, i.e. each agent is administered at a different time, as well as simultaneous administration of these agents. "Co-administration" or "combination therapy" is not intended to embrace incidental overlap of separate therapies wherein each agent is administered for a purpose distinct from the other. Simultaneous administration may be accomplished by, e.g. administering a male one or more formulations comprising both agents or administering one or more separate formulations each comprising a single agent.

The term "pharmaceutically acceptable salt" refers to a salt prepared from a pharmaceutically acceptable non-toxic inorganic or organic acid. Inorganic acids include hydrochloric, hydrobromic, hydroiodic, nitric, sulfuric, and phosphoric. Organic acids include, but are not limited to, aliphatic, aromatic, carboxylic, and sulfonic organic acids including formic, acetic, propionic, succinic, benzoic camphorsulfonic, citric, fumaric, gluconic, isethionic, lactic, malic, mucic, tartaric, paratoluenesulfonic, glycolic, glucuronic, maleic, furoic, glutamic, benzoic, anthranilic, salicylic, phenylacetic, mandelic, embonic (pamoic), methanesulfonic, ethanesulfonic, pantothenic, benzenesulfonic, stearic, sulfanilic, alginic, and galacturonic acid. A preferred salt is the citrate salt.

### Antiestrogen + Androgen

Accordingly, in one embodiment, the present disclosure provides a method for increasing testosterone levels in a male by sequential administration of an antiestrogen (or salt thereof) and an androgen. Sequential co-administration of the antiestrogen (or salt thereof) and the androgen may comprise consecutive alternating rounds of treatment in which an androgen (or an antiestrogen) is administered for a period of time after which administration of the androgen (or antiestrogen) is discontinued, followed by administration of an antiestrogen (or androgen) for a period of time after which administration of the antiestrogen (or androgen) is discontinued and so on. Suppression of pituitary gonadotropins may persist after androgen replacement therapy is discontinued and therefore testosterone levels may remain depressed below pre-treatment levels for a period of time after cessation of androgen replacement therapy. In a related aspect, the present disclosure provides a method for alleviating the post-treatment effects of androgen replacement therapy by administering an antiestrogen (or salt thereof) less than one week, less than two weeks, less than three weeks, less than one month, or less than two, three, four, five or six months after discontinuing androgen replacement therapy.

In another embodiment of the disclosure, a method is provided for increasing testosterone levels in a male by simultaneous administration of an antiestrogen (or salt thereof) and an androgen, in which case the antiestrogen and the androgen may be delivered in the same or in different formulations.

Co-administration (sequential or simultaneous) of an antiestrogen (or salt thereof) and an androgen may be accomplished by any appropriate route, including injection (preferably intramuscular), oral, transdermal (e.g. patches), topical (e.g. gels and creams) and transmucosal (e.g. buccal) administration. An antiestrogen (or salt thereof) and testosterone may be administered by the same or different route. For example, the antiestrogen may be administered orally and testosterone may be administered transdermally, transmucosally or by intramuscular injection. Alternatively, for example, the antiestrogen and testosterone may both be administered orally or topically.

Androgens may be administered by any appropriate route; however, injection (preferably intramuscular), oral, transdermal (e.g. patches), topical (e.g. gels and creams) and transmucosal (e.g. buccal) administration are preferred routes. When administered, the androgen is in a pharmaceutically acceptable formulation and is present in a pharmacologically effective amount (i.e. the dose of androgen is sufficient to elevate testosterone levels in the male to therapeutic levels of at least 300 ng/dL, preferably at least 350 ng/dL, more preferably at least 400 ng/dL, even more preferably at least 500 ng/dL). Representative androgens for use according to the methods of the disclosure include testosterone, testosterone esters (e.g. enanthate or cypionate), and 17-alkylated androgens such as fluoxymesterone and methyltestosterone.

In one embodiment of the disclosure, the androgen is testosterone. Testosterone may formulated as a gel for topical application. Examples of topical testosterone formulations include topical gels such as Androgel® (Solvay Pharmaceuticals, Inc., Marietta, GA, USA), Testim® (Auxilium Pharmaceuticals, Inc., Malvern, PA, USA) and Fortesta® (Endo Pharmaceuticals, Chadds Ford, PA, USA). When administered in the form of a topical gel, the dosage of testosterone is preferably between 10mg and 100mg and the gel is applied to skin daily, for example at a dose of about 50, 75 or 100 mg/day. Alternativley, testosterone may be formulated as a patch for transdermal administration such as Androderm® (Watson Laboratories, Corona, CA, USA) or Testoderm® (Alza Corp., Palo Alto, CA, USA). When administered in the form of a transdermal patch, the dosage of testosterone is preferably between 2mg to 5mg/day and the patch is applied to the skin once or twice per day. Testosterone may also be in a formulation suitable for buccal administration such as Striant® (Actient Pharmaceuticals, Lake Forest, IL, USA). Buccal formulations are preferably administered to the upper gum at a dosage of about 30 mg applied twice daily. Testosterone may also be formulated as a pellet for subcutaneous implantation such as Testopel® (Slate Pharmaceuticals, Durham, NC, USA) which are administered at a dosage of 150 to 450mg testosterone every 3-6 months. Alternatively, testosterone may be in a formulation suitable for oral administration (i.e. to be ingested).

In another embodiment of the disclosure, the androgen is a testosterone ester such as, testosterone undecanoate (e.g. Nebido®), testosterone cypionate (e.g. Depo®-Testosterone), or testosterone enanthate (e.g. Delatestryl®). The testosterone ester may also be a priopionate, phenylpropionate, isocaproate or decanoate ester, and may be formulated as a blend of all (e.g. Sustanon®) or a subset of these esters. Testosterone esters are preferably provided in oil in a formulation suitable for intramuscular injection. Dosage regimens vary according to individual patient, testosterone ester and diagnosis. Generally between 50 to 400mg of the testosterone ester is administered every two to four weeks (or 10 to 21 days); however, longer acting testosterone esters (e.g. the undecanoate ester) may be administered less frequently such as every three months. Testosterone esters may also be formulated for oral administration (i.e. for ingestion) such as Andriol® (testosterone undecanoate) which is recommended to be administered 2-5 times per day at a dosage of 80-200 mg/day or OriTex® (testosterone undecanoate).

In another embodiment of the disclosure, the androgen is a 17-alkylated androgen such as fluoxymesterone (sold under the trade name Halotestin®) or methyltestosterone (sold under the trade name Android®) in which case the androgen is preferably administered orally.

In a preferred embodiment of the disclosure, no additional therapeutic agents are co-administered with an antiestrogen (or salt thereof) and an androgen according to the methods described herein. In one aspect of the disclosure, aromatase inhibitors are not co-administered with an antiestrogen and an androgen in practicing the methods.

The antiestrogen, or pharmaceutically acceptable salt thereof, when co-administered with an androgen, is present in an amount sufficient to improve one or more fertility parameters in a male undergoing testosterone replacement relative to the same parameter(s) in the absence of the antiestrogen. Males undergoing testosterone therapy may exhibit LH and FSH levels of 0.5 U/L or less. The antiestrogen (or salt thereof) is preferably administered in an amount effective to elevate one or both of these gonadotropins to levels of at least 3 U/L, at least 4 U/L, at least 5 U/L, at least 6 U/L, at least 7 U/L, at least 8 U/L, at least 9 U/L, at least 10 U/L, at least 11 U/L, at least 12 U/L, at least 13 U/L, at least 14 U/L, or even at least 15 U/L. The antiestrogen may be co-administered with an androgen for the duration of the androgen replacement therapy or may be co-administered with the androgen for a portion of the androgen replacement therapy.

Male mammals that may benefit from the superposition of antiestrogen administration on androgen replacement therapy include any male mammal with a need or desire to elevate his testosterone levels. Preferably, the male mammal is a human male. The male may or may not be naive to androgen replacement therapy prior to being co-administered an androgen and an antiestrogen. Thus, co-administration of an antiestrogen, preferably trans-clomiphene, may be superposed on testosterone replacement therapy in a male with low (less than ∼300 ng/dl) or low-normal (300-400 ng/dl) serum testosterone levels regardless of the underlying etiology. Human males with human immunodeficiency virus or acquired immunodeficiency syndrome may benefit from testosterone therapy in order to treat muscle wasting, depression and/or fatigue. Accordingly, in a related aspect of the disclosure, a human male with human immunodeficiency virus or acquired immunodeficiency syndrome is co-administered an antiestrogen and an androgen in order to treat muscle wasting, depression and/or fatigue.

### Antiestrogen + Aromatase Inhibitor

In several embodiments of the disclosure, a combination therapy comprising sequential or simultaneous administration of an effective amount of one or more aromatase inhibitors and an antiestrogen or pharmaceutically acceptable salt is provided. By "aromatase inhibitor" it is meant non-steroidal and steroidal compounds that inhibit the enzyme aromatase thereby preventing the conversion of androgens to estrogens, preferably those which inhibit aromatase activity *in vitro* with an IC₅₀ value of less than 10⁻⁵ M as well as their pharmaceutically acceptable salts. Aromatase inhibitors of the disclosure useful in the combination therapy include, anastrozole, letrozole, exemestane, vorozole, formestane, fadrozole, aminoglutethimide, testolactone, 4-hydroxyandrostenedione, 1,4,6-androstatrien-3,17-dione and 4-androstene-3,6,17-trione. Preferred aromatase inhibitors of the invention include selective inhibitors such as anastrazole, letrozole, and vorozole. Aromatase inhibitors for use in the combination therapy can be formulated, together with a pharmaceutically acceptable carrier, into pharmaceutical compositions which can be administered by any acceptable delivery method such as oral (i.e. ingestion), transmucosal (e.g. buccal or rectal), transdermal or parenteral (e.g. intravenous, subcutaneous, intramuscular) administration. An effective amount of an aromatase inhibitor for use in the combination therapy herein described can be determined by a doctor in view of the physical condition of the male and the specific aromatase inhibitor to be used, but a typical dose will range from 1 mg to 100 mg, preferably 1 to 50 mg, 1 to 30 or 1 to 15 mg per dose. Dosages of aromatase inhibitor may be administered daily or may be administered periodically such as every other day, every third day, weekly, every other week, or monthly.

In a related embodiment of the disclosure, a method for preventing or ameliorating an antiestrogen-dependent increase in estrogen levels in a male mammal with secondary hypogonadism is provided comprising co-administering an effective amount of one or more aromatase inhibitors with an antiestrogen or salt thereof to said male mammal. In a preferred embodiment of the disclosure, the male mammal is a human male with a BMI of at least 30, at least 31, at least 32, at least 33, at least 34, at least 35, at least 36, at least 37, at least 38, at least 40, at least 41, at least 42, at least 43, at least 44, or even at least 45.

The antiestrogen, or pharmaceutically acceptable salt thereof, and aromatase inhibitor are each present in an amount effective to elevate testosterone in a male mammal to whom the combination of agents is administered, preferably to levels of at least 300 ng/dl, which can be determined by any known method of measuring and monitoring testosterone levels in a male mammal. In preferred embodiments of the disclosure, the effective amounts of aromatase inhibitor and antiestrogen are less than the effective amount of each agent when administered separately. In other preferred embodiments, the effect of the combination of aromatase inhibitor and antiestrogen on increasing testosterone levels is greater than the predicted effect of the agents when administered separately.

### Antiestrogens

Antiestrogens for use in the combination therapies described herein may be in the form of solids, such as tablets or filled capsules or liquids such as solutions suspensions, emulsions, elixirs or capsules filled with the same, all for oral use. The compositions may also be in the form of sterile injectable solutions or emulsions for parenteral (including subcutaneous) use. Such pharmaceutical compositions and unit dosage forms thereof may comprise ingredients in conventional proportions.

Within the context of the disclosure, it has been discovered that therapeutic effects may persist after cessation of antiestrogen administration, presumably due to a correction/resetting of the hypothalamic-pituitary-testicular axis; accordingly, the antiestrogen (or salt thereof) may be administered on a daily basis or may be administered intermittently, such as every other day, every third day, weekly, biweekly or even monthly. Where the antiestrogen is administered intermittently, an interval of 3-30 days or more may be present between consecutive doses and these intervals may vary throughout the administration period. For example, the antiestrogen may be administered at a dosing regime of 3, 4, 5, 6, 7, 8, 9, 10, 11, 12, 13, 14, 15, 16, 17, 18, 19, 20, 21, 22, 23, 24, 25, 26, 27, 28, 29, 30 or more days between consecutive administrations. Within the context of the invention, the frequency of administration of trans-clomiphene or pharmaceutically acceptable salt thereof is preferably daily for at least seven consecutive days, after which the frequency of administration of trans-clomiphene is intermittently such as every other day, every third day, weekly, bi-weekly or monthly. Within the context of the invention, in another preferred embodiment, the frequency of administration oftrans-clomiphene is daily or every other day.

The antiestrogen may be present at any dose at which the antiestrogen is therapeutically effective. In one aspect of the disclosure, the antiestrogen is administered at a dose of from about 1 to 200 mg, preferably from about 5 to about 100 mg (although the determination of optimal dosages is with the level of ordinary skill in the art). In one aspect of the invention, the antiestrogen is administered at a dose of from 5 to 100 mg (although the determination of optimal dosages is with the level of ordinary skill in the art). In a preferred embodiment of the invention, the antiestrogen dose may also be from 12.5 to 50 mg (e.g. 12.5, 25 or 50 mg). Within the disclosure the antiestrogen dose may also be from about 12.5 to about 50 mg (e.g. 12.5, 25 or 50 mg). Antiestrogen dosages are preferably (but not necessarily) administered as part of a dosage regimen designed to preserve the diurnal pattern of gonadotropin secretion, while increasing the amplitude of both FSH and LH. For example, according to FIG. 1 a dosage of antiestrogen may be administered in a pharmaceutical formulation that would give rise to peak serum testosterone levels at around 8 a.m. After administration of the composition serum testosterone levels may be measured as described above and dosages may be altered to achieve a sufficient increase in the serum testosterone levels to achieve the desired physiological results associated with normal testosterone described above.

By "antiestrogen" it is meant a compound that prevents estrogens from expressing their effects on estrogen dependent target tissues consequently antagonizing a variety of estrogen-dependent processes. In all cases, antiestrogens useful in the practice of the instant invention are those capable of blocking the negative feedback exerted by normal estrogens on the pituitary leading to increases in LH and FSH. In men, these increased levels of gonadotropins stimulate the Leydig cells of the testes and result in the production of higher testosterone levels. Antiestrogens useful in the practice of the instant invention may be pure antiestrogens or may have partial estrogenic action as in the case of the selective estrogen receptor modulators (SERMs) which exhibit antiestrogenic properties in some tissues and estrogenic tissues in others. In a preferred aspect of the invention, trans-clomiphene is a preferred antiestrogen for use in the methods described herein, preferably in a form substantially free of cis-clomiphene (e.g. in a composition comprising about 100% w/w trans-clomiphene and about 0% w/w cis-clomiphene as active agent).

Pure antiestrogens of the disclosure include, without limitation: RU 58,688, described in Van de Velde et al, Ann. NY Acad. Sci., 761(3): 164-175 (1995); 13-methyl-7-[9-(4,4,5,5,5-pentafluoropentylsulfinyl)nonyl]-7,8,9, 11 , 12, 13 , 14, 15, 16,17-decahydro-6H-cyclopenta[a]-phenanthrene-3,17-diol (ICI 182,780/fulvestrant) and other compounds described in EP 0138504; N-butyl-11-[(7R,8S,9S,13S,14S,17S)-3,17-dihydroxy-13-methyl-6,7,8,9,11,12,14,15,16, 17-decahydrocyclopena[a]phenanthren-7-yl]-N-methyl-undecanamide (ICI 164,384), described in Wakeling and Bowler, J. Endocrin., 112:R7-R110 (1987); (#)-7-pivaloyloxy-3-(4'pivaloyloxyphenyl)-4-methyl-2-(4" (2"piperidinoethoxy)phenyl)-2H-benzopyran (EM-800/SCH 57050) and other compounds described in WO 96/26201; (2S)-3-(4-hydroxyphenyl)-4-methyl-2-[4-[2-(1-piperidyl)ethoxy]phenyl]-2H-chromen-7-ol (EM-652/SCH 57068).

SERMs useful in the methods of the disclosure include, without limitation, triphenylalkylenes such as triphenylethylenes, which include: 2-[4-(1,2-diphenylbut-1-enyl)phenoxy]-N,N-dimethyl- ethanamine (tamoxifen) and other compounds described in U.S. Patent No. 4,536,516; Trans-4-(1-(4-(2-dimethylamino)ethoxy)phenyl)-2-phenyl-1-butenyl)phenol (4-hydroxytamoxifen) and other compounds described in U. S. Patent No. 4,623,660, 1-[4'-dimethylaminoethoxy)phenyl]-1-(3'-hydroxyphenyl)-2-phenylbut-1-ene (droloxifene) and other compounds described in U.S. Patent No. 5,047,431, 2-[p-[(Z)-4-chloro-1,2-diphenyl-1-butenyl]phenoxy]-N,N-dimethylethylamine (toremifene) and other compounds described in U.S. Patent Nos. 4,696,949, 5,491,173 and 4,996,225, (E)- 1 - (2-(4-(I-(4-enyl)-phenoxy)-ethyl)-pyrrol idinone (idoxifene) and other compounds described in U.S. Patent No. 4,839,155, clomiphene and both its isomers; and compounds described in U.S. Patent Nos. 4,696,949 and 5,491,173 and 6,576,645,

SERMS useful in the methods of the disclosure also include, benzothiphene derivatives such as: [6-hydroxy-2-(4-hydroxyphenyl)-benzothiophen-3-yl]-[4-[2-(1-piperidinyl)ethoxy)phenyl]-methanone (raloxifene) and other compounds described in U.S. Patent Nos. 4,418,068 and 5,393,763, LY353381; and LY335563 and other compounds described in WO 98/45286, WO 98/45287 and WO 98/45288; benzopyran derivatives such as: (#)-7-pivaloyloxy-3-(4'pivaloyloxyphenyl)-4-methyl-2-(4"-(2"piperidinoethoxy)phenyl)-2H-benzopyran (EM 800 /SCH 57050) and other compounds described in WO 96/26201; (2S)-3-(4-hydroxyphenyl)-4-methyl-2-[4-[2-(1-piperidyl)ethoxy]phenyl]-2H-chromen-7-ol (EM 652); naphthalene derivatives such as: Cis-6-phenyl-5-[4-(2-pyrrolidin-1-yl-ethoxy)-phenyl]-5,6,7,8-tetrahydronaphthalen-2-ol (lasofoxifene/CP 336,156) and other compounds described in U.S. Patent No. 5,552,412; 3,4-dihydro-2-(p-methoxyphenyl)-1-naphthyl-p-[2-(1-pyrrolidinyl)ethoxy]phenyl ketone (trioxifene/LY133314) and other compounds described in U.S. Patent No. 4,230,862, and 1-(4-Substituted alkoxy)benzyl)naphthalene compounds such as those described in U.S. Patent No. 6,509,356, chromans such as 3,4-trans-2,2-dimethyl-3-phenyl-4-[4-(2-(2-(pyrrolidin-1-yl)ethoxy)phenyl]-7-methoxychroman (levormeloxifene) and other compounds described in WO 97/25034, WO 97/25035, WO 97/25037 and WO 97/25038; and 1-(2-((4-(-methoxy-2,2, dimethyl-3 -phenyl-chroman-4-yl)-phenoxy)-ethyl)-pyrrolidine (centchroman) and other compounds described in U.S. Patent No. 3,822,287,

Other SERMs of the disclosure include, without limitation, the compounds described in U.S. Patent Nos. 6,387,920, 6,743,815, 6,750,213, 6,869,969, 6,927,224, 7,045,540, 7,138,426, 7,151,196, and 7,157,604,

Further non-limiting antiestrogens of the disclosure include: 6a-chloro-16α-methyl-pregn-4-ene-3,20-dione (clometherone); 6-chloro-17-hydroxypregna-1,4,6-triene-3,20-dione (delmadinone); 1-[2-[4-[1-(4-methoxyphenyl)-2-nitro-2-phenylethenyl]phenoxy]ethyl]-pyrrolidine (nitromifene/CN-55,945-27); and 1-[2-[p-(3,4-Dihydro-6-methoxy-2 -phenyl- 1-naphthyl)phenoxy] ethyljpyrrolidine (nafoxidene) .

Further non-limiting antiestrogens of the disclosure include indoles such as those disclosed in J. Med. Chem., 33:2635-2640 (1990), J. Med. Chem., 30:131-136 (1987), WO 93/10741, WO 95/17383, WO 93/23374 and U.S. Patent Nos. 6,503,938 and 6,069,153.

Further non-limiting antiestrogens of the disclosure include 2-[3-(1-cyano-1-methylethyl)-5-(1H-1 ,2,4-triazol- 1 -ylmethyl)phenyl]-2-methyl-propanenitrile (anastrozole) and other compounds described in EP 0296749; 6-Methylenandrosta-1,4-diene-3,17-dione (exemestane) and other compounds described in U.S. Patent No. 4,808,616,; 4-[(4-cyanophenyl)-(1,2,4-triazol-1-yl)methyl]benzonitrile (letrozole) and other compounds described in U.S. Patent No. 5,473,078, incorporated herein by reference; 1-[4'-dimethylaminoethoxy)phenyl]-1-(3'-hydroxyphenyl)-2-phenylbut-1-ene (droloxifene) and other compounds described in U.S. Patent 5,047,431,; 2α,3α,-Epithio-5α,-androstan-17β-ol (epitiostanol); 2α,3α,-Epitio-5α,-androstane-17β-yl-1-methoxycyclopentyloxy (mepitiostane); 4-[(2Z,4Z)-4-(4-hydroxyphenyl)hexa-2,4-dien-3-yl]phenol (cycladiene) and other compounds described in U.S. Pat. Nos. 2,464,203 and 2,465,505,; CI-680 described in Unlisted Drugs, 28(10): 169(0) (1976); CI-628 described in Unlisted Drugs, 26(7): 106(1) (1974); 13-ethyl-17α,-ethynl-17β-hydroxygona-4,9,1-trien-3-one (R2323); diphenol hydrochrysene and erythyro-MEA both described in Geynet, et ah, Gynecol. Invest. 3(1):2-29 (1972); 1-[1-chloro-2,2-bis(4-methoxyphenyl)ethenyl]-4-methoxy-benzene (chlorotrianisene) described in Merck Index, 101 ed., #2149; 1-[4-(2-Diethylaminoethoxy)phenyl]-1-phenyl-2-(p-anisyl)ethanol (ethamoxytriphetol) described in Merck Index, 101 ed., #3668; and 2-p-Chlorophenyl-1-[p-(2-diethylaminoethoxy)phenyl]-1-p-tolylethanol (triparanol) and other compounds described in U.S. Patent No. 2,914,562,.

Still other antiestrogens of the disclosure include, (2e)-3-(4-((le)-1,2-diphenylbut-1-enyl)phenyl)acrylic acid (GW5638), GW7604 and other compounds described in Wilson et al, Endocrinology, 138(9):3901-3911 (1997) and WO 95/10513; 1-[4-(2-diethylaminoethoxy)phenyl]-2-(4-methoxyphenyl)-1-phenyl-ethanol (MER-25), N,N-diethyl-2-[4-(5-methoxy-2-phenyl-3H-inden-1-yl)phenoxy]ethanamine hydrochloride (U-11,555A), 1-[2-[4-(6-methoxy-2-phenyl-3,4-dihydronaphthalen-1-yl)phenoxy]ethyl]pyrrolidine hydrochloride (U-Il, 100A), ICI-46,669, 2-[4-[(Z)-1,2-diphenylbut-1-enyl]phenoxy]-N,N-dimethyl-ethanamine; 2-hydroxypropane-1,2,3-tricarboxylic acid (ICI-46,474) and other compounds described in Terenius et al., Gynec. Invest., 3:96-107 (1972); 2-Hydroxy-6-naphthalenepropionic acid (allenolic acid); [4-[(4-acetyloxyphenyl)-cyclohexylidene-methyl]phenyl]acetate (cyclofenyl/ICI-48213); [6-hydroxy-2-(4-hydroxyphenyl)benzothiophen-3-yl]-[4-[2-(1-piperidyl)ethoxy]phenyl]methanone (keoxifene); 4-[(Z)-1-[4-(2-dimethylaminoethoxy)phenyl]-2-(4-propan-2-ylphenyl)but- 1 - enyl]phenol (DP-TAT-59/miproxifene); (1RS,2RS)-4,4'-diacetoxy-5,5'-difluoro-(1-ethyl-2-methylene)di-m-phenylenediacetate (acefluranol); 6-hydroxy-2-(p-hydroxyphenyl)-benzo(b)thien-3-yl[2-(1-pyrrolidinyl)-ethoxyphenyl]ketone (LY-117018); and [6-hydroxy-2-(4-hydroxy-phenyl)benzo(b)thien-3-yl]-[4-(2-(1-piperdinyl)-ethoxy)phenyl]methanone (LY-156758).

Still other antiestrogens of the disclosure include, without limitation: non-steroidal estrogen receptor ligands such as those described in U.S. Patent Nos. 5,681,835, 5,877,219, 6,207,716, 6,340,774 and 6,599,921; steroid derivatives such as those described in U.S. Patent No. 4,659,516, 7α-11-aminoalkyl-estratrienes such as those described in WO 98/07740; 11-β-halogen-7α-substituted estratrienes such as those described in WO 99/33855; 17α-alkyl-17β-oxy-estratrienes such as those described in U.S. Patent Application No. 10/305,418; 2-phenyl-1-[4-(2-aminoethoxy)-benzyl]-indoles such as those described in U.S. Patent No. 7.132,417; 4-fluoroalkyl-2h-benzopryans such as those described in U.S. Patent No. 6,844,336; (4-(2-(2-aza-bicyclo[2.2.1]hept-2-yl)-ethoxy)-phenyl)-(6-hydroxy-2-(4-hydroxy-phenyl)-benzo[b]thiop hen-3-yl)-methanone and other benzothiophenes described in WO 95/10513 and U.S. Patent No. 4,133,814; 2-phenyl-1-[4-(2-aminoethoxy)-benzyl] -indoles such as those described in U.S. Patent No. 5,998,402; 3- [4- (2- Phenyl-Indole- 1- ylmethyl) Phenyl]- Acrylamides and other compounds described in U.S. Patent No. 5,985,910; 2-phenyl-1-[4-(amino-1-yl-alk-1-ynyl)-benzyl]-1H-indol-5-ols and other compounds described in U.S. Patent Nos. 5,780,497 and 5,880,137; steroids such as those described in U.S. Patent Nos. 6,455,517, 6,548,491, 6,747,018 and 7,041,839; Di-(3'-hydroxyphenyl)-alkane compounds such as those described in U.S. Patent No. 4,094,994; phenol derivatives such as those described in U.S. patent No. 4,751,240; 2,3-diaryl-2H-1-benzopyran analogs such as those described in Saeed et al., J. Med. Chem., 33:3210-3216 (1990) and Sharma et al, J. Med. Chem. 33:3216-3229 (1990); and benzofuran and triarylfuran analogs such as those described in Durani et al, J. Med. Chem., 32: 1700-1707 (1989).

### Subjects

An antiestrogen (or salt thereof) may be co-administered with an androgen or aromatase inhibitor to male mammals with low or low-normal testosterone levels to treat the testosterone deficiency per se (i.e. to treat primary or secondary hypogonadism) or to treat one or more disorders related to low testosterone level. Primary and secondary hypogonadism each describe and are partially defined by low testosterone levels; however, LH levels are elevated in the case of primary hypogonadism and are low or low-normal in the case of secondary hypogonadism. Examples of disorders related to low testosterone which may be treated by the combination therapies herein described, include without limitation, all aspects of metabolic syndrome (e.g. elevated cholesterol, triglycerides), type 2 diabetes mellitus, lipodystrophy or osteoporosis. Accordingly, in several aspects of the disclosure, a method is provided for treating a disorder associated with low testosterone selected from the group consisting of metabolic syndrome or a symptom thereof, type 2 diabetes mellitus, lipodystrophy and osteoporosis by co-administering an antiestrogen or pharmaceutically acceptable salt thereof and an androgen or aromatase inhibitor to a male in need of such treatment. Low testosterone is associated with a number of physiologic symptoms including, without limitation, decreased libido, depressed mood, erectile dysfunction, decrease in muscle mass, and reduction in cognitive function. Accordingly, in one aspect of the disclosure, hypogonadal (e.g. secondary hypogonadal) males with one or more of these symptoms are co-administered an antiestrogen, preferably trans-clomiphene, and an androgen or aromatase inhibitor to alleviate the symptoms. Accordingly, in one aspect of the invention, hypogonadal (e.g. secondary hypogonadal) males with one or more of these symptoms are co-administered an trans-clomiphene with aromatase inhibitor to alleviate the symptoms. An association between body mass index (BMI) and secondary hypogonadism has recently been identified and high BMI appears to be a primary underlying etiology in the disorder, particularly in relatively young males. Moreover, males with high BMI and secondary hypogonadism are at increased risk for developing metabolic syndrome and ultimately type 2 diabetes mellitus. Accordingly, secondary hypogonadal males with a BMI of at least 25, at least 30, at least 35 or at least 40 (e.g. BMI of 25-30, 30-35, 35-40) may be co-administered an androgen or aromatase inhibitor and an antiestrogen in order to treat the secondary hypogonadism and/or in order to treat or prevent an associated disorder such as metabolic syndrome or type 2 diabetes mellitus.

In another embodiment of the disclosure a pharmaceutical composition is provided comprising an androgen and an antiestrogen or pharmaceutically acceptable salt thereof and at least one pharmaceutically acceptable carrier. Also provided is a kit comprising an androgen and an antiestrogen or pharmaceutically acceptable salt thereof which may be formulated in the same or in different compositions along with instructions for use according to any method described herein.

In a separate embodiment of the disclosure a pharmaceutical composition is provided comprising an aromatase inhibitor and an antiestrogen or pharmaceutically acceptable salt thereof and at least one pharmaceutically acceptable carrier. Also provided is a kit comprising an aromatase inhibitor and an antiestrogen or pharmaceutically acceptable salt thereof which may be formulated in the same or in different compositions along with instructions for use according to any method described herein.

### REFERENCE EXAMPLE 1

### Effects of Clomids on Serum Testosterone and Cholesterol in Male Baboons

Adult, male, Baboons were given 1.5 mg/kg of Clomid, Enclomid (*trans-*Clomid) or Zuclomid (*cis*-Clomid) for 12 consecutive days. The samples analyzed were sera taken on the day of first treatment before being given test article (day 0), after 12 days of treatment (day 12) and 7 days after the last treatment (end or wash-out).

### 1. Effects on Body Weight and Serum LH, FSH, PRL and Testosterone

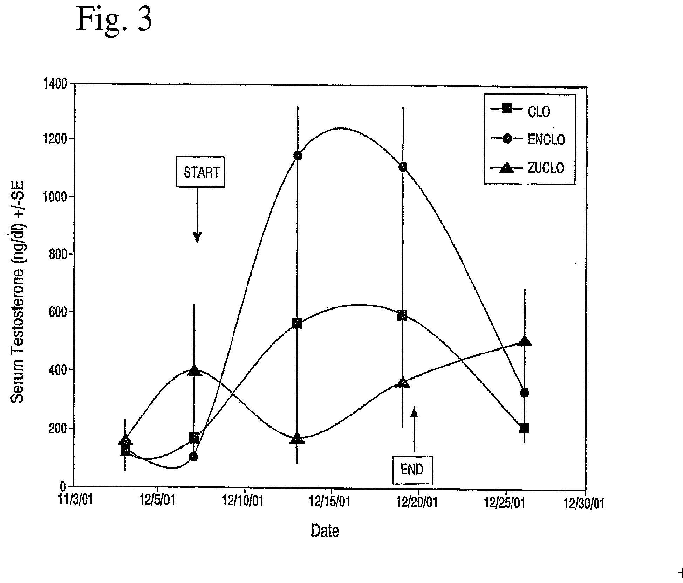

There were significant increases in total serum testosterone in the group receiving Enclomid. See Table 1. There were no differences among groups in the baseline period or at day 0. There were also no differences among the three groups 7 days after treatment (the washout period). However, Enclomid produced higher levels of testosterone compared to Clomid and Zuclomid on day 6 (p = 0.03 and p = 0.00002 respectively) and compared to Zuclomid on day 12 (p = 0.047). Zuclomid clearly did not raise total serum testosterone to any extent. Compared to the animals receiving Enclomid, the animals receiving Clomid exhibited more variable total testosterone levels on day 6 and later as judged by their coefficients of variations. When we looked at the time course of the effects (FIG. 3), we determined that only Enclomid significantly and statistically raised total serum testosterone on days 6 and 12 compared with either baseline or day 0 values. Moreover, cessation of Enclomid treatment, resulted in a significant drop in the level of total serum testosterone between day 12 and day 18 (washout). This indicates that Enclomid is readily cleared from the circulation consistent with the metabolic clearance seen for Enclomid in humans. Enclomid was clearly better and more consistent than Clomid itself and Zuclomid was ineffective.

**Table 1 - Serum Testosterone Levels (ng/dl)**

| Group | ID | baseline 12/3/01 | 0 day 12/7/01 | 6 days 12/13/01 | 12 days 12/20/01 | wash-out 12/26/01 |
|---|---|---|---|---|---|---|
| CLO | 7500 | 79.01 | 76.15 | 940.97 | 891.5 | 150.9 |
| | 9012 | 97.55 | 305.24 | 585.92 | 555.6 | 316.3 |
| | 9097 | 158.06 | 102.94 | 151.12 | 318.9 | 143.6 |
| | **mean** | **111.5** | **161.4** | **559.3** | **588.7** | **203.6** |
| | SD | 41.3 | 125.2 | 395.6 | 287.7 | 97.7 |
| ENCLO | 7223 | 64.57 | 74.96 | 1223.8 | 633.6 | 307.2 |
| | 8021 | 166.86 | 133.59 | 1128.2 | 1466 | 399.2 |
| | 8369 | 170.45 | 106.47 | 1081.1 | 1166 | 271 |
| | **mean** | **134.0** | **105.0** | **1144.4** | **1088.5** | **325.8** |
| | SD | 60.1 | 29.3 | 72.7 | 421.6 | 66.1 |
| ZUCLO | 7438 | 124.84 | 210.4 | 137.51 | 314.5 | 359.7 |
| | 8292 | 104.66 | 67.37 | 169.98 | 406.1 | 860.5 |
| | 10098 | 282.29 | 904.82 | 227.95 | 353.0 | 274.1 |
| | **mean** | **170.6** | **394.2** | **178.5** | **357.9** | **498.1** |
| | SD | 97.3 | 448.0 | 45.8 | 46.0 | 316.8 |
| | ANOVA | **p = 0.61** | p = 0.43 | p = 0.007 | **p = 0.57** | p = 0.256 |
| | K-W | p = 0.56 | **p = 0.84** | **p** = **0.051** | p = 0.079 | **p = 0.252** |

There were no changes in serum LH or FSH. The ratio of total serum testosterone to LH followed the same pattern as total serum testosterone, suggesting a lack of dependence (data not shown). There was also no change in body weight during the 12 day study. There was a decrease in serum prolactin (PRL) during the study in the group receiving Enclomid, suggesting an effect of antiestrogen that has been described in part (Ben-Jonathan and Hnasko, 2001) and expected on the basis of the fact that as men age, testosterone declines and Prolactin increase (Feldman *et al.*, 2002).

### 2. Effects on Cholesterol Levels

Treatment with Enclomid tended to decrease serum cholesterol and Zuclomid tended to increase the same parameter. Preliminary analysis indicated that the changes in cholesterol levels were not statistically significant and that the changes were within the normal range. Due to the observed trend for the two isomers to demonstrate opposite effects on cholesterol levels over a short period of time, further analysis was conducted.

Detailed analysis indicated that Enclomid resulted in an 8% decrease in serum cholesterol levels. Conversely, treatment with Zuclomid resulted in a 22% increase in serum cholesterol levels. Treatment with Clomid resulted in a slight increase in serum cholesterol levels. The opposite effect of Enclomid and Zuclomid on serum cholesterol levels is not unexpected given that the isomers have, alternatively, estrogen agonist or antagonist activity. These results indicate that Enclomid may be used for treating patients with high cholesterol levels. These results also indicate that Enclomid may be more benign than Zuclomid with respect to serum cholesterol if used chronically for increasing testosterone levels.

### 3. Effects on Clinical Chemistry Parameters

The mean values for each parameter did not differ among the three groups for any test parameter at the beginning of the study as determined by ANOVA or by the Kruskal-Wallis test. All groups exhibited normal values at each parameter except for (1) serum sodium; a related calculated parameter, anionic gap, which were low for all nine baboons throughout the trial; (2) serum glucose; and (3) BUN which were high on day 0 for the group which would be treated with Enclomid. On day 12 of treatment and 7 days after treatment (washout), there were no differences among groups for any parameter except anionic gap that showed that the Clomid and Zuclomid groups had lower values than the Enclomid group. The values of serum sodium and anionic gap appear to be anomalies associated with this group of baboons.

There were substantive effects on the red blood cell population with Enclomid and Zuclomid and on hematocrit with Zuclomid. All the compounds lower the mean cell hemoglobin concentration (MCHC) either at day 0 or at the endpoint. With no change in mean cell hemoglobin (MCH) and an increase in the mean cell volume (MCV), the lowering of MCHC is predictable. Although testosterone might be expected to raise hematocrit, only Zuclomid treatment, which did not increase total serum testosterone, demonstrated a statistical difference. Clearly, men in a clinical trial that uses Zuclomid should be monitored for the characteristics of their red blood cell population. Enclomid would be predicted to have less of an effect.

There appears to be a clear effect of 12-day Enclomid treatment on platelets although the values found stayed within the normal range. One thing to consider here is the sexual dimorphism in platelet counts between male and female baboons (279 for males vs. 348 for females). This is likely to be due to hormones. Since the Enclomid group demonstrated increased testosterone, the lowering of the platelet count could be secondary to the change in testosterone in this group. Moreover, treatment with Enclomid pushed the platelet count to its normal male level from a day 0 level that was the high end of the normal range for this group. Enclomid would not necessarily predict a deleterious effect on platelets.

All the Clomids tested had effects on the white blood cell (WBC) population, the most striking was that of Enclomid on raising the counts of lymphocytes and eosinophiles. The effects are not as straightforward as they would seem to be. There appears to be a strong effect of Enclonud on lowering the per cent of granulocytes in the blood. The effects are very strong after the 7-day washout period when the values are decreased below the normal range. (This time course could reflect the relatively long time required to affect change the WBC population.) There is little sexual dimorphism in baboons with respect to the white blood cell populations, so the effects are more likely to be due to the compound itself than changes in testosterone. However, when we look at the calculated count of granulocytes using the WBC count, we find no differences in granulocyte count due to any compound. Concomitantly, it is the lymphocyte story that is the most interesting. Both the count and per cent lymphocytes in the population increase with Enclomid treatment. Whereas the mean values of per cent lymphocytes remain in the normal range, given the trend for an increase in WBC count, the net effect is an increase in lymphocyte count with Enclomid. This eosinophil result is analogous. There is a clear implication for treating men who have low lymphocytes, such as men who are HIV-positive. Since Enclomid is unlikely to lower lymphocytes based on this result, a case could be made for its use in the population of men with AIDS. These individuals are often treated with agents that are intended to raise testosterone due to the wasting effects of disease. Low liver and kidney toxicity and favorable effects on cholesterol and lipids are also highly favored attributes for any medication intended for use HIV-positive men who are already compromised by their disease.

The increase in serum glucose with Clomid or Zuclomid was within the normal range. In the case of Enclomid where the mean serum glucose values were high on day 0, there were no increases with treatment. There was no evidence that Enclomid would have a deleterious effect on blood glucose.

No clearly adverse effects on liver function are apparent as judged by the enzymes AST and ALT. The trend in these values was a decrease with treatment. An increase in the level of enzymes in the serum would indicate liver damage. ALT/SGPT was out of range low at the end of the study for the Clomid group although the differences over the treatment period were not statistically significant. The changes with Enclomid and Zuclomid were within the normal range. AST is depressed in pregnancy; thus the action of an estrogen agonist such as Zuclomid in lowering the marginal AST level could be rationalized. Alkaline phosphatase (ALP) is also found in the liver and is elevated various disease states. The lowering of ALP argues further against hepatic damage. There were no changes in serum albumin, also a liver product. A strong suppression of serum albumin over an extended time period could contribute to free serum steroid hormone levels in humans although a more important role is played by sex hormone binding globulin. As a bottom line, none of the compounds could be linked to liver damage on the basis of the parameters assayed.

Osteoblastic activity and diseases of the bone are accompanied by high serum ALP values. ALP was not elevated following Zuclomid treatment and was decreased in value following Enclomid treatment. The trends would predict a more benign result for the use of Enclomid compared to Zuclomid.

Although BUN and BUN/creatinine were altered during the study in the Clomid and Enclomid groups, the lack of a definitive change in creatinine argues against renal dysfunction. A loss of glomerular filtration capacity would result in an increase in BUN. Decreased BUN occurs in humans due to poor nutrition (not likely in a controlled setting), or high fluid intake (presumably accompanied by edema). Also, despite an increase in total serum testosterone between day 0 and Day 12 with Enclomid, there were no differences between serum creatinine values, arguing against an increase in muscle mass over this short time interval.

Serum sodium levels were lower than reference values for all animals throughout the study. Serum carbon dioxide was higher than reference values on day 12 for the Clomid and Zuclomid groups. Serum anion gap was lower for all animals throughout the study, paralleling the sodium results. Enclomid raised this parameter towards normal values. The electrolyte imbalances detected in the test animals throughout all treatment periods remains elusive but might be part of the same fluid derangement phenomenon suggested by the BUN results.

The foregoing results indicate that Enclomid is more effective than Clomid or Zuclomid at enhancing total serum testosterone. Zuclomid is clearly not effective and that deficiency limits any use of Clomid for hypogonadism, particularly since the Zuclomid component of Clomid would predominate in the circulation over time given its longer half-life.

Enclomid appeared to be relatively benign in all aspects when compared to Zuclomid and, often, even Clomid. This is particularly true when consideration is given to the trend of Enclomid to lower cholesterol, and liver enzymes as opposed to Zuclomid's trend to raise the same parameters. The surprising trend for Enclomid to raise the lymphocyte count may be useful for men with AIDS if it can be shown the CD4+ subpopulation of lymphocytes is not lowered or is enhanced.

### EXAMPLE 2

### Method for Increasing Testosterone Level in Men Using Trans-clomiphene and Mixtures of Trans-clomiphene and Cis-clomiphene at Ratios Greater Than 1

Prior to administration of *trans*-clomiphene, blood samples are taken from subject males and testosterone levels are measured using methodologies described for example in Matsumoto, et al. Clin. Endocrinol. Metab. 56; 720 (1983) (incorporated herein by reference). Sex hormone binding globulin (SHBG), both free and bound to testosterone, may also be measured as described for example in Tenover et al. J. Clin. Endocrinol. Metab. 65:1118 (1987) which describe measurement of SHBG by both a [³H] dihydrotestosterone saturation analysis and by radioimmunoassay. Non-SHBG-bound testosterone levels (bioavailable testosterone) are also measured for example according to Tenover et al. J. Clin. Endocrinol and Metab. 65:1118 (1987). See also Soderguard et al. J. Steroid Biochem 16:801 (1982) incorporated herein by reference.

Patients are given daily dosages of 1.5 mg/kg clomiphene, wherein the ratio of *trans-*clomiphene to *cis*-clomiphene is greater than 1. Patients are monitored for testosterone levels such that the dosage amount and dosage frequency may be adjusted to achieve therapeutic levels of testosterone in the patient.

### EXAMPLE 3

### Comparison of Androxal™ to Androgel®

A placebo controlled challenge study was conducted at the Advanced Biological Research, Inc. (ABR) Clinical Research Center in Hackensack, New Jersey to compare orally administered Androxal™ (trans-clomiphene) to Androgel® in hypogonadal men. Androgel® (Solvay Pharmaceuticals, Inc.) consists of a cream that administers exogenous testosterone in a transdermal matrix.

The study enrolled 62 hypogonadal men with testosterone levels less than 300 ng/dl (normal 298-1034 ng/dl) that were randomized into 6 different arms, three doses of Androxal™ (12.5 mg, 25 mg, and 50 mg), placebo, and both high and low doses of Androgel®. Half of the men in each of the Androxal™ and placebo arms were randomized into cohorts that underwent in-clinic sessions on days 1 and 14 to determine pharmacokinetic parameters for Androxal™ as well as cyclical changes in testosterone. The placebo and Androxal™ doses were administered in a double blind fashion. The Androgel® cream was administered in an open label fashion. Half of the Androgel® patients underwent in-clinic sessions similar to the other patients in the study. Following the two week drug exposure patients were followed for an additional seven to ten days to determine the status of their testosterone levels. There were no side effects noted in either the Androxal™ or Androgel® arms of the study that were different than placebo.

### 1. Effects on Testosterone Levels

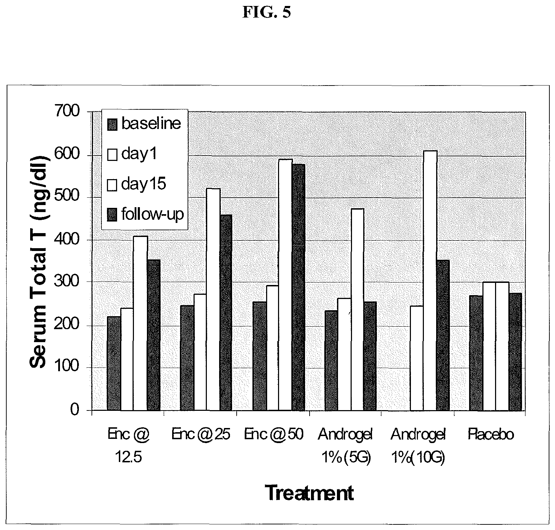

All doses of Androxal™ or Androgel® produced statistically significant changes in testosterone from baseline testosterone levels (FIG. 5). The low, mid and high doses of Androxal™ achieved mean increases of 169, 247, and 294 ng/dl respectively, while those of Androgel® 5G, the lowest approved dose, and Androgel® 10G, the highest approved dose, produced changes from baseline that were 212 and 363 ng/dl. These values were statistically indistinguishable from those changes achieved with Androxal™. This inability to show differences between Androxal™ and Androgel® appears to result from the highly variable results found when Androgel® is used. For example the 50 mg dose of Androxal™ raised mean total testosterone to 589±172 ng/dl after 15 days, a coefficient of variation (CV) of 29% and similar to the placebo group (36%). On the other hand Androgel® 5G and 10G yielded mean total testosterone values 473±289 ng/dl and 608±323 ng/dl, CV's of 61% and 53% respectively.

After 14 days of Androxal™ therapy all doses were associated with a total testosterone diurnal pattern similar to the placebo group, i.e. a morning peak, a mid-day trough and a rise overnight. Without being bound by theory, this pattern may be due to the mode of action of Androxal™, which appears to be mediated through effects on the hypothalamic-pituitary axis as shown below. The diurnal pattern for men on Androgel® was nearly flat. However, spikes in total testosterone for Androgel® were associated with dosing and often exceeded the normal high level of 1,034 ng/dl. Certain individuals on Androgel® 10G were able to achieve peak levels of total testosterone of over 2500 ng/dl.

Interestingly, the level of serum total testosterone in the follow-up period (i.e., 7-10 days after cessation of daily oral treatment) unexpectedly remained high after treatment with Androxal™. In addition, the serum total testosterone levels were significantly higher at the highest dose of Androxal™ compared to the high dose of AndroGel® 1% (p = 0.017, t-test).

### 2. Effects on LH and FSH Levels

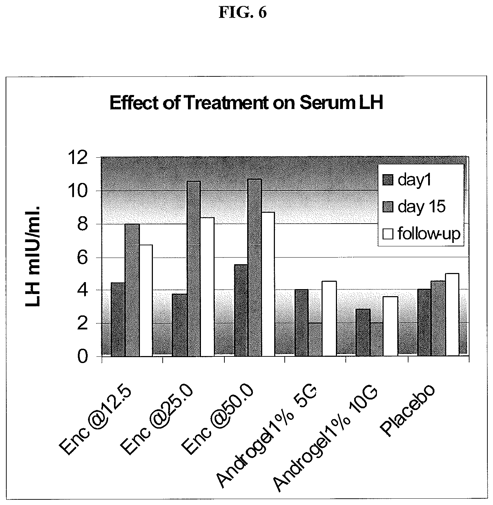

Treatment with Androxal™ produced a statistically significant increase in the serum levels of LH in the hypogonadal male subjects (FIG. 6). As in the case of total serum testosterone there was an unexpected continuation in the level of serum LH in the follow-up period (i.e., 7-10 days after cessation of daily oral treatment) where those levels remained high for the three doses of Androxal™. By comparison, treatment with AndroGel® initially decreased LH and after cessation there was an apparent rebound towards pre-treatment levels.

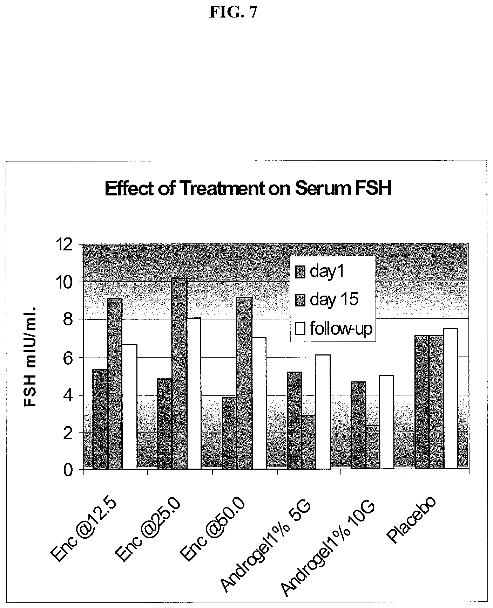

Treatment with Androxal™ also produced a statistically increase in the serum levels of FSH in the hypogonadal male subjects (FIG. 7). The pattern of increasing FSH is similar to that seen in the case of LH, that is, all doses of Androxal™ boosts serum FSH which remains high during the follow-up period whereas AndroGel® suppresses the level of serum FSH and cessation of treatment allows serum FSH to rebound towards concentrations more similar to pre-treatment levels.

### 3. Effects on Other Clinical Chemistry Parameters

The effect on serum dihydroxytestosterone (DHT) levels were also measured. Men on Androxal™ experienced a favorable shift in their DHT to total testosterone. For example men on the 50 mg dose of Androxal™ experienced a DHT/TT ratio of 0.83 as compared to the placebo group ratio of 1.07. By contrast the DHT/TT ratio for either of the Androgel® groups was >1.5. The results indicate that men on Androgel® were gaining DHT faster than total testosterone. Thus the normal levels of DHT was disrupted relative to testosterone in men on Androgel® therapy.

Results of clinical chemistry parameters also indicated, unexpectedly, that men on Androxal™ experienced a non-dose dependent reduction in triglycerides. The reduction in triglycerides averaged a decrease of 19.1% after two weeks of therapy. This compared to a 5.9% reduction for the placebo group and increases of 0.3% and 22% for the Androgel® 5G and 10G respectively.

### 4. Discussion

Based on this study we infer a number of potential advantages for Androxal™ as a potential therapy. Androxal™ appears to raise total testosterone into the normal range in a highly consistent manner without abnormally high spikes in serum testosterone. In addition, the use of transclomiphene to treat men that suffer secondary hypogonadism offers a new approach that potentially could offset one of the major side effects of exogenous therapies such as Androgel®. Exogenous therapies provide negative feedback thereby shutting down FSH and LH production. FSH is an essential reproductive hormone and in the male stimulates spermatogenesis. Long term exposure to exogenous testosterone, as a result of its effects on FSH production, causes a reduction in sperm synthesis, leading to the potential for transient infertility due to low sperm counts and therefore a resulting shrinkage of the testis, since the volume of the testis is related to the level of spermatogenesis within the seminiferus tubules. The increase in FSH levels also indicates that Androxal™ may be used to treat infertility in males, including hypogonadal males. Moreover, the extended affects of Androxal™ on serum testosterone, FSH and LH levels indicate that Androxal™ may be administered with altered dosages or scheduling, allowing perhaps even non-daily or episodic treatment.

### EXAMPLE 4

### Three Dose Comparison of Androxal to Androgel

A clinical trial was conducted to compare the effects of three doses of trans-clomiphene (administered orally once per diem) to the effects of Androgel® used per manufacturer's instructions. Sixty men with testosterone levels not exceeding 350 ng/dl were enrolled in the study and randomized to one of four parallel arms: (i) 6.25 mg Androxal (ii) 12.5 mg Androxal (iii) 25 mg Androxal or (iv) Androgel. The average age of men in the study was 53.1 (10.1) and their average body mass index (BMI) was 31.8 (6.1). The men were dosed for 6 weeks and then followed for an additional 7 days after cessation of treatment. Subjects in all arms visited the clinic every 2 weeks for assessments. Twenty-four hour assessments were made at baseline before dosing commenced and after 6 weeks of treatment, at which point testosterone and LH were assessed hourly. The results are provided below at Table 2:

**Table 2**

| Dose | Baseline T ng/dl (stdev) | Week 6 T ng/dl (stdev) | Follow-up T ng/dl (stdev) | ITT LOCF ng/dl (stdev) | 24 hr avg. T ng/dl (stdev) | 24hr T < 300ng/dl @ Week 6 | Any T > 1100 ng/dl @ Week 6 |
|---|---|---|---|---|---|---|---|
| 6.25 mg | 247 (75.6) | 392 (154.2) | 341 (150.7) | n=15 | n=12 | 4 out of 12 | 0 out of 12 |
| | | | | 402 (159.3) | 392 (152.8) | | |
| 12.5 mg | 312(110.5) | 495 (170.4) | 437 (188.2) | n=14 | n=9 | 2 out of 10 | 0 out of 10 |
| | | | | 493 (163.9) | 461 (129.2) | | |
| 25 mg | 248 (114.8) | 577 (133.4) | 612 (125.4) | n=16 | n=13 | 0 out of 13 | 0 out of 13 |
| | | | | 541 (159.0) | 587 (142.1) | | |
| Androgel® | 293 (117.5) | 452 (243.0) | 242.3 (89.3) | n=14 | n=13 | 2 out of 13 | 3 out of 13 |
| | | | | 452 (243.1) | 544 (230.1) | | |

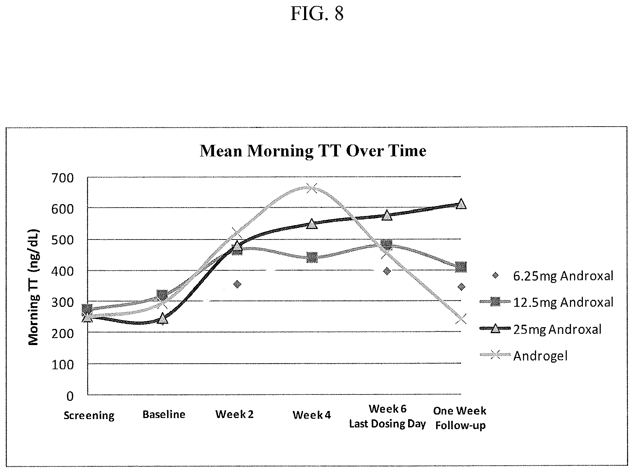

All Androxal arms exhibited improved morning testosterone compared to baseline after the seven day follow-up and there was a clear dose response in the Androxal arms. The legacy effect noted with Androxal a week after cessation of treatment was quite strong reaching statistically significant treatment differences for the high and mid doses of Androxal compared to Androgel (p<0.0001 for the 25 mg dose and p=0.003 for the 12.5 mg dose). The comparison to the 6.25 mg dose was nearly significant, p=0.056. Seven days after cessation of treatment, the drug should no longer be onboard and the continued improvement may relate to a resetting of the hypothalamo-pitituary-testes axis. Figure 8 displays the mean total testosterone (TT) observed over the course of the study including the follow-up one week after dosing has stopped.

The topical gel arm exhibited morning testosterone levels numerically worse than the baseline values, presumably due to the suppressive effects of exogenous testosterone on pituitary function. As in previous studies, topical testosterone significantly suppressed FSH and LH, in many cases to castration levels.

### EXAMPLE 5

### Comparison of Androxal to Testim

A placebo-controlled clinical trial was conducted to compare the effects of trans-clomiphene (administered orally once per diem) to the effects of Testim® (1% topical testosterone gel, 5 g applied daily at a dose of 50 mg testosterone) in secondary hypogonadal men with moderate to severe dysfunction following a three month dosing period. In order to meet the criteria for enrollment, men were required to exhibit a morning testosterone of < 250 ng/dl on two separate assessments separated by at least 10 days and must have been naive to testosterone treatment. Over 900 men were screened to achieve a total of 108 men who met all criteria and were enrolled in the study. All 108 men enrolled in the study satisfied the requirement of at least one dose and one visit in which efficacy measures could be assessed. The subjects were randomized into four groups: (i) placebo; (ii) 12.5mg Androxal; (iii) 25mg Androxal; and (iv) Testim. There was no statistical difference among the groups in testosterone at baseline. At the end of the three month dosing period a statistically significant increase in final median morning testosterone levels was observed in all three active arms. See Table 3, below.

**Table 3: Median Morning Testosterone Data**

| | Androxal 12.5 mg (n=26) | Androxal 25 mg (n=30) | Placebo (n=24) | Testim (n=28) |
|---|---|---|---|---|
| Mean Age | 49.2 | 49.6 | 52.7 | 51.5 |
| Mean BMI | 32.4 | 31.8 | 30.9 | 32.7 |
| Baseline T | 202 | 202 | 220 | 207 |
| Final T | 432 | 416 | 196 | 393 |
| P value vs. Placebo | <0.00001 | <0.00001 | | 0.0002 |

There was no statistical difference observed among the active arms; all three active arms were highly statistically different than the placebo arm. Medians were used for the statistical analysis due to the non-standard distribution of the Testim arm. Two of the 28 men in the Testim arm exceeded the normal maximum level of testosterone (1100 ng/dl) while no men in the Androxal arms exceeded the normal maximum level. In the Androxal arms 69.9% of men were in the normal range for morning testosterone levels (300-1100 ng/dl) versus 57.1% of the men in the Testim arm.

Sperm counts were assessed at baseline and after dosing was completed. Spermatogenesis is approximately a 64 day cycle and detection of early changes after 3 months of treatment would be expected. At baseline, the median values of all four arms were above the generally accepted lower level of sperm counts for normal males (20 million sperm per ml). At the end of the three month dosing period there were no statistically significant changes in sperm counts for the two Androxal groups and the placebo group. The Testim group exhibited a statistically significant reduction (p=0.003) in median sperm counts to 6.2 million sperm/ml, well below the normal range and into a range defined as oligospermia. The median sperm count for the men in the combined Androxal arms was 93.8 million sperm/ml (p value=0.001 compared to Testim). The men in the placebo arm exhibited a median sperm count of 110.8 million sperm/ml and it was also significantly different than Testim (p=0.005). The drug was generally well tolerated at both doses compared to placebo. No drug related serious adverse effects occurred that led to discontinuation.

### EXAMPLE 6

### Comparison of the Effects of Androxal to Testim on Spermatogenesis

A clinical trial was conducted to determine the effects of trans-clomiphene (administered orally once per diem) on spermatogenesis when administered for 6 months to males with secondary hypogonadism (testosterone<300 ng/dl; LH<15 IU/ml) who had previously been treated for between 6 months and 2 years with topical exogenous testosterone. The mean age of the subjects was 46 years and mean body weight was 233 pounds. 13 Subjects were randomized into two treatment groups that received (i) Androxal 12.5mg/day or (ii) Testim applied daily according to manufacturer's suggestions, with 11 subjects finishing the study. Subjects were required to discontinue topical exogenous testosterone for at least 30 days prior to receiving study medication. Effects of the treatment on sperm parameters associated with fertility as well as on FSH and LH levels were evaluated following 3 and 6 months of therapy and one month after cessation of treatment. Semen analysis included semen volume, total sperm count, sperm concentration, percent motility and percent normal morphology. There were 6 laboratory visits including a screening (VI), baseline (V2) and post first dose visits at 1 month (V3), 3 months (V4) and 6 months (V5) as well as a one month post therapy follow-up visit (V6).

After subjects were washed out of their previous testosterone treatment for a period of three weeks, study subjects showed morning mean total testosterone levels of 165 +/- 66 ng/dL. After 3 months of treatment there was a statistically signficant rise in mean serum testosterone in men receiving Androxal and Testim that was sustained for another 3 months with continued treatment. Androxal also significantly increased both LH and FSH while men on Testim had significantly lower levels of these gonadotropins.

Androxal consistently elevated sperm counts in 7 out of 7 men on Androxal at 3 months and 6 out of 6 men on Androxal at 6 months with a range of sperm concentrations from 75-334 million/ml, well within the normal range, and sperm concentrations remained within the normal range at follow-up. Testim, on the other hand, resulted in sperm counts below 20 million (the level below which a man would be considered infertile) for all 5 men at 3 months. At 6 months, 3 of the 5 Testim treated men remained below the 20 million level. Three of the five Testim subjects had sperm counts of essentially zero for all visits. The difference in sperm count was significant between the Androxal and Testim groups at all time points.

Testim use resulted in significantly lower motile sperm concentration (sperm concentration X sperm motility) than Androxal. Androxal tended to increase motile sperm concentration between V2 and V4. Testim use also resulted in significantly lower motile sperm counts than Androxal.

Subjects treated with Androxal compared to Testim demonstrated an increase in sperm concentration with significant differences seen at Visits 4, 5 and 6. No such increase was observed in the Testim arm. Moreover, sperm obtained from subjects in the Androxal group were more morphologically normal than sperm obtained from subjects in the Testim group and were significantly different at Visit 5.

Testim use elevates serum levels in total testosterone into normal ranges in males with secondary hypogonadism and also produced an apparent negative feedback in the hypothalamus/pituitary axis resulting in decreased LH and FSH production. The observed decrease of LH and FSH blood levels appears to result in azoospermia or oligospermia which would lead to lowered fertility rates. Androxal use also maintains total testosterone levels in the normal ranges and also increases LH and FSH levels resulting in renewed or continued sperm production. Both drugs were well tolerated by all subjects during the 6 months of drug therapy.

## Claims

1. A combination of trans-clomiphene or a pharmaceutically acceptable salt thereof, and an aromatase inhibitor which is selected from anastrozole, letrozole and exemestane, for use in treating secondary hypogonadism or a symptom thereof in a human male with secondary hypogonadism and a body mass index of at least 30.

2. A combination for use according to claim 1, wherein the pharmaceutically acceptable salt is a citrate salt.

3. A combination for use according to claim 1, wherein trans-clomiphene or pharmaceutically acceptable salt thereof and the aromatase inhibitor are for separate administration.

4. A combination for use according to claim 1, wherein trans-clomiphene or pharmaceutically acceptable salt thereof and the aromatase inhibitor are for sequential or simultaneous use.

5. A combination for use according to claim 1, wherein a dosage of trans-clomiphene is from 5-100 mg, preferably 12.5 mg, 25 mg or 50 mg.

6. A combination for use according to claim 5, wherein the frequency of administration of trans- clomiphene is daily or every other day.

7. A combination for use according to claim 1, wherein the symptom of secondary hypogonadism is selected from reduction of muscle mass, limitation of body performance, reduction of bone density, reduction of libido, reduction of potency, metabolic syndrome, type 2 diabetes, osteoporosis and infertility.

8. A combination for use according to any preceding claim, wherein the frequency of administration of trans-clomiphene or pharmaceutically acceptable salt thereof is daily for at least seven consecutive days, after which the frequency of administration of trans-clomiphene is intermittently such as every other day, every third day, weekly, bi-weekly or monthly.

9. A kit comprising an aromatase inhibitor which is selected from anastrozole, letrozole and exemestane and trans-clomiphene or a pharmaceutically acceptable salt thereof.

10. A combination for use according to any one of claims 3-9 wherein the salt thereof is a citrate salt.

## Patentansprüche

1. Kombination von trans-Clomiphen oder eines pharmazeutisch akzeptablen Salzes davon und eines Aromatase-Inhibitors, welcher aus Anastrozol, Letrozol und Exemestan ausgewählt ist, zur Verwendung in der Behandlung des sekundären Hypogonadismus oder eines Symptoms davon bei einem männlichen Menschen mit sekundärem Hypogonadismus und einer Körpermassenzahl von mindestens 30.

2. Kombination zur Verwendung nach Anspruch 1, wobei das pharmazeutisch akzeptable Salz ein Citratsalz ist.

3. Kombination zur Verwendung nach Anspruch 1, wobei trans-Clomiphen oder ein pharmazeutisch akzeptables Salz davon und der Aromatase-Inhibitor zur getrennten Gabe vorgesehen sind.

4. Kombination zur Verwendung nach Anspruch 1, wobei trans-Clomiphen oder ein pharmazeutisch akzeptables Salz davon und der Aromatase-Inhibitor zur sequenziellen oder gleichzeitigen Verwendung vorgesehen sind.

5. Kombination zur Verwendung nach Anspruch 1, wobei eine Dosis von trans-Clomiphen 5-100 mg, vorzugsweise 12,5 mg, 25 mg oder 50 mg, ist.

6. Kombination zur Verwendung nach Anspruch 5, wobei die Frequenz der Gabe von trans-Clomiphen täglich oder jeden zweiten Tag ist.

7. Kombination zur Verwendung nach Anspruch 1, wobei das Symptom des sekundären Hypogonadismus aus der Verringerung der Muskelmasse, Einschränkung der körperlichen Leistungsfähigkeit, Verringerung der Knochendichte, Verringerung der Libido, Verringerung der Potenz, dem metabolischen Syndrom, Typ-2 Diabetes, Osteoporose und Unfruchtbarkeit ausgewählt ist.

8. Kombination zur Verwendung nach irgendeinem vorhergehenden Anspruch, wobei die Frequenz der Gabe von trans-Clomiphen oder eines pharmazeutisch akzeptablen Salzes davon für mindestens sieben aufeinanderfolgende Tage täglich ist, nach welchen die Frequenz der Gabe von trans-Clomiphen periodisch ist, beispielsweise jeden zweiten Tag, jeden dritten Tag, wöchentlich, alle zwei Wochen oder monatlich.

9. Satz, umfassend einen Aromatase-Inhibitor, welcher aus Anastrozol, Letrozol und Exemestan ausgewählt ist, und trans-Clomiphen oder ein pharmazeutisch akzeptables Salz davon.

10. Kombination zur Verwendung nach irgendeinem der Ansprüche 3-9, wobei das Salz davon ein Citratsalz ist.

## Revendications

1. Combinaison de trans-clomiphène ou d'un sel pharmaceutiquement acceptable de celui-ci, et d'un inhibiteur de l'aromatase choisi parmi l'anastrozole, le létrozole et l'exémestane, pour une utilisation dans le traitement de l'hypogonadisme secondaire ou d'un symptôme de celui-ci chez un sujet humain de sexe masculin présentant un hypogonadisme secondaire et un indice de masse corporelle d'au moins 30.

2. Combinaison pour une utilisation selon la revendication 1, dans laquelle le sel pharmaceutiquement acceptable est un sel de citrate.

3. Combinaison pour une utilisation selon la revendication 1, dans laquelle le trans-clomiphène ou le sel pharmaceutiquement acceptable de celui-ci et l'inhibiteur de l'aromatase sont destinés à une administration séparée.

4. Combinaison pour une utilisation selon la revendication 1, dans laquelle le trans-clomiphène ou le sel pharmaceutiquement acceptable de celui-ci et l'inhibiteur de l'aromatase sont destinés à une utilisation séquentielle ou simultanée.

5. Combinaison pour une utilisation selon la revendication 1, dans laquelle une posologie de trans-clomiphène est comprise entre 5 et 100 mg, de préférence de 12,5 mg, 25 mg ou 50 mg.

6. Combinaison pour une utilisation selon la revendication 5, dans laquelle la fréquence d'administration du trans-clomiphène est journalière ou d'un jour sur deux.

7. Combinaison pour une utilisation selon la revendication 1, dans laquelle le symptôme de l'hypogonadisme secondaire est choisi parmi la réduction de masse musculaire, la limitation de performances corporelles, la réduction de densité osseuse, la réduction de libido, la réduction de puissance sexuelle, le syndrome métabolique, les diabètes de type 2, l'ostéoporose et l'infertilité.

8. Combinaison pour une utilisation selon l'une quelconque des revendications précédentes, dans laquelle la fréquence d'administration du trans-clomiphène ou du sel pharmaceutiquement acceptable de celui-ci est journalière pendant au moins sept jours consécutifs, après quoi la fréquence d'administration du trans-clomiphène est intermittente, comme tous les deux jours, tous les trois jours, de façon hebdomadaire, toutes les deux semaines, ou de façon mensuelle.

9. Kit comprenant un inhibiteur de l'aromatase choisi parmi l'anastrozole, le létrozole et l'exémestane et le trans-clomiphène ou un sel pharmaceutiquement acceptable de celui-ci.

10. Combinaison pour une utilisation selon l'une quelconque des revendications 3 à 9, dans laquelle son sel est un sel de citrate.
